(19) Europäisches Patentamt
European Patent Office
Office européen des brevets

(11) Publication number: **0 042 288**
**B1**

(12) **EUROPEAN PATENT SPECIFICATION**

(45) Date of publication of patent specification: **28.05.86**

(51) Int. Cl.[4]: **G 01 S 7/52,** G 01 S 15/89, A 61 B 10/00

(21) Application number: **81302677.0**

(22) Date of filing: **16.06.81**

(54) **Ultrasonic imaging apparatus.**

(30) Priority: **16.06.80 JP 81799/80**

(43) Date of publication of application:
**23.12.81 Bulletin 81/51**

(45) Publication of the grant of the patent:
**28.05.86 Bulletin 86/22**

(84) Designated Contracting States:
**DE FR GB**

(56) References cited:
**WO-A-80/01537**
**US-A-3 156 110**
**US-A-3 425 031**
**US-A-3 523 275**
**US-A-4 167 879**

(73) Proprietor: **FUJITSU LIMITED**
**1015, Kamikodanaka Nakahara-ku**
**Kawasaki-shi Kanagawa 211 (JP)**

(72) Inventor: **Murakami, Keiischi**
**1259-8, Kamikodanaka Nakahara-ku**
**Kawasaki-shi Kanagawa 211 (JP)**
Inventor: **Amemiya, Shinichi**
**2-1, Fujigaoka Midori-ku**
**Yokohama-shi Kanagawa 227 (JP)**
Inventor: **Miyazaki, Junji**
**5-4, Ibukino Midori-ku**
**Yokohama-shi Kanagawa 227 (JP)**
Inventor: **Yanashima, Tadahiko**
**3245-5, Fujisawa**
**Fujisawa-shi Kanagawa 251 (JP)**
Inventor: **Iida, Atsuo**
**Myorenji-House A-702 1-19 Nakatehara Kohoku-ku**
**Yokohama-shi Kanagawa 222 (JP)**
Inventor: **Shimura, Takaki**
**29-44, Tsurukawa 4-chome Machida-shi**
**Tokyo 194-01 (JP)**
Inventor: **Miwa, Hirohide**
**6-7-10, Miyazaki Takatsu-ku**
**Kawasaki-shi Kanagawa 213 (JP)**

**EP 0 042 288 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(72) Inventor: **Midorikawa, Norio**
**1-5, Tsuchihashi 7-chome Takatsu-ku**
**Kawasaki-shi Kanagawa 213 (JP)**
Inventor: **Iwashita, Nobushi**
**4-1-808, Yokodai Isogo-ku**
**Yokohama-shi Kanagawa 235 (JP)**

(74) Representative: **Sunderland, James Harry et al**
**HASELTINE LAKE & CO Hazlitt House 28**
**Southampton Buildings Chancery Lane**
**London WC2A 1AT (GB)**

**Description**

This invention relates to ultrasonic imaging apparatus.

Ultrasonic imaging technology is widely employed for obtaining images of the internal structure of objects such as the human body.

In ultrasonic imaging technology, an ultrasonic wave is transmitted to an object, a reflected wave or a transmitted wave is received from the object, and then, in a case in which the object is the human body, a tomographic image, for example, of the tissue structure of the body is obtained on the basis of the received wave. Ultrasonic imaging has advantages in that it is nondestructive and is less dangerous than X-ray imaging.

Ultrasonic imaging apparatus using the so-called pulse reflection method is widely known. This method relies upon the reception of a reflected wave from an object. The operation of such ultrasonic imaging apparatus using the pulse reflection method will be explained, as an example.

An ultrasonic wave pulse in the frequency range of about 1 MHz to 10 MHz is transmitted to an object from a piezoelectric element of a transducer, an echo pulse (reflected wave) which is reflected as a result of a mismatching of impedances at a boundary, between two types of tissue in the human body for example having different acoustic impedances, within the object is received, and information relating to the location of the areas of such mismatching can be obtained by displaying such a received wave.

This is called A mode operation.

A tomographic image can be obtained by taking the following steps:— the location or angle of ultrasonic wave pulse transmission is sequentially shifted in accordance with the location of a tomographic area and pieces of location information pertaining to areas of mismatching are combined for display on the basis of the reflected waves corresponding to respective transmitted pulses.

This is called B mode operation.

In such ultrasonic imaging apparatus, one ultrasonic wave pulse is transmitted after an immediately preceding ultrasonic wave pulse. The time interval between one transmitted pulse and the immediately preceding pulse is sufficient for the one ultrasonic wave pulse to be transmitted (to the object) and then to return as an echo pulse. The transmission interval for ultrasonic wave pulses is resultingly subject to a limitation; the interval must be long enough for completion of reception of an echo pulse.

Since the propagation velocity of ultrasonic waves in the tissues of the human body is about 1500 m/sec, the period of from transmission of a pulse to reception of an echo pulse is 2L/1500 (sec), where the depth of the boundary responsible for the reflection giving rise to the echo pulse is L, and the minimum pulse transmission interval is therefore 2L/1500 (sec).

The number of scanning lines that can be obtained during one second is limited to 1500/2L (lines). For example, when L is 0.2 m, this number of scanning lines is 3750 (lines) and therefore it is not possible to obtain a sufficient number of scanning lines when a tomographic image is required within a very short period (for example, 0.1 sec).

Moreover, the limitation on the number of scanning lines has an effect upon the display of a tomographic image on a CRT display unit—.

Since a frame rate of about 30 frames/sec is necessary for displaying motion within an object without the display appearing to flicker, the number of scanning lines in each frame is limited to 25/L (lines). In other words, when L=0.2 m, the number of scanning lines is 125 lines/frame, which is only 1/4 the number of scanning lines per frame commonly used in television receivers. This limitation on the number of scanning lines can result in very coarse display of tomographic images, reducing the imaging effect.

Thus, existing ultrasonic wave imaging apparatus has disadvantages in that a sufficient number of scanning lines cannot be obtained, it is difficult accurately to image momentary or instantaneous conditions of tissues in a human body, for example, and only a very rough or crude display of a tomographic image can be obtained, due to the inherent limitation of the propagation velocity of ultrasonic waves, when imaging dynamic tissues such as the heart etc, of the human body for example.

US—A—3 156 110 discloses ultrasonic apparatus for detecting and imaging the internal structure of an object by transmitting ultrasonic pulses into the object and receiving echoes of the pulses. The apparatus provides a colour display of the frequency dependent characteristics of the internal structure of the object. Three transducers, driven by respective pulse generators, transmit respective pulsed ultrasonic beams into the medium. The three ultrasonic beams have different carrier frequencies. The three transducers receive echoes from the medium. Respective receivers are connected to the three transducers, tuned respectively to the three carrier frequencies. Signals from the receivers are used to determine the intensities of respective "primary" colours of the colour display.

In an effort to overcome the problems mentioned above (insufficient number of scanning lines, rough or crude display), the inventors of the present invention have proposed improved ultrasonic imaging apparatus in the specification of International Patent Application No. PCT/JP80/00015 entitled "Ultrasonic Diagnostic System"—corresponding to European Appln No 80900270.2 (publication No 32513).

According to that specification, ultrasonic waves of different frequencies are simultaneously transmitted from a plurality of transducers, a reflected signal or a transmitted signal from an object is received, and then the reflected waves or transmitted waves corresponding to the respective different frequencies are obtained from the received signal using an electrical filter.

This technique presents simultaneously a several times greater amount of information relating to the internal structure of an object than previous apparatus.

On the other hand, when the human body is the object to be investigated, attenuation in the object generally increases as frequency increases.

For this reason, when employing the above-mentioned technique, it is desirable that the differences between the different frequencies are kept as small as possible. That is, the frequency $f_1$ of the one transmitted wave and the frequency $f_2$ of another transmitted wave are very close to one another.

However, when the frequencies $f_1$ and $f_2$ are very close, it is difficult to provide sufficient separation using only an electrical filter.

For this reason, even after a received signal has passed through an electrical filter, so-called crosstalk occurs. In other words, the signal of frequency $f_2$ still affects the signal of frequency $f_1$.

An embodiment of the present invention can provide ultrasonic imaging apparatus which is capable of substantially preventing crosstalk between a plurality of ultrasonic wave frequencies.

An embodiment of the present invention can provide ultrasonic imaging apparatus which is capable of substantially preventing crosstalk without the use of a very accurate and expensive electrical filter.

An embodiment of the present invention can provide ultrasonic imaging apparatus which can offer simultaneously a large amount of accurate information on the internal structure of an object.

An embodiment of the present invention can provide ultrasonic imaging apparatus which is suitable for investigating internal tissues of the human body.

An embodiment of the present invention can provide ultrasonic imaging apparatus which is capable of utilising the transmission and reception frequency characteristics of transducers to provide a filtering function.

According to the present invention there is provided ultrasonic imaging apparatus, for imaging the internal structure of an object by transmitting an ultrasonic wave into the object and receiving an acoustic wave from the object, the apparatus including at least two transmit/receive transducer means, for transmitting ultrasonic waves and receiving acoustic waves, having respective different frequency characteristics and functioning as frequency filters by virtue of their own frequency characteristics,

each of said transducer means having a transmission characteristic and a reception characteristic both including a transducer resonance frequency, the transmission characteristic extending above the resonance frequency beyond the reception characteristic and the reception characteristic extending below the resonance frequency beyond the transmission characteristic,

a first transmission circuit connected to a first of the said transmit/receive transducer means, for causing it to transmit an ultrasonic wave of a first frequency,

a first reception circuit connected to the first transmit/receive transducer means,

a second transmission circuit, connected to a second of the said transmit/receive transducer means, for causing it to transmit an ultrasonic wave of a second frequency, higher than the first frequency,

a second reception circuit connected to the second transmit/receive transducer means, and

an electrical filter provided in the first transmission circuit or in the second reception circuit, the filter characteristic of the electrical filter being determined in dependence upon the transmission characteristic of the first transmit/receive transducer means and the reception characteristic of the second transmit/receive transducer means, so as to reduce crosstalk between the transducer means.

For further complete enforcement of crosstalk elimination, an electrical filter can also be provided in the second transmission circuit and the first reception circuit.

Thus, whilst the frequency characteristics of transducers are used to provide a filter function, remaining elements not compensated for by this filter function can then be compensated for by the electrical filter.

An embodiment of this invention can provide for an improvement in ultrasonic imaging apparatus for imaging the internal structure of an object by utilising ultrasonic waves, and particularly can provide for an improvement in ultrasonic imaging apparatus which transmits an ultrasonic wave to an object and receives a reflected wave returning from the object or a wave transmitted through the object.

Reference is made, by way of example, to the accompanying drawings, in which:—

Figures 1 and 2 are respective schematic diagrams for assistance in explanation of the basic principles of ultrasonic imaging, for a better understanding of the present invention,

Figure 3 is a time chart for further explanation of the present invention,

Figure 4 is a graphical illustration of the frequency characteristic of an ultrasonic wave transducer as used in an embodiment of the present invention,

Figure 5 is a graphical illustration of frequency characteristics of two ultrasonic wave transducers as used in an embodiment of the present invention,

Figures 6 and 7 are respective graphical illustrations of frequency characteristics, for explanation of the effects of the use of an electrical filter in accordance with embodiments of the present invention,

Figure 8 is a graphical illustration of frequency characteristics for explanation of the effects of the use of a band-pass filter in accordance with an embodiment of the present invention,

Figure 9 illustrates frequency characteristics in a case in which a higher frequency component is compensated for the effects of greater attenuation, in an embodiment of the present invention,

Figures 10A and 10B illustrate characteristics of respective electrical filters which can be employed in embodiments of the present invention,

Figure 11 is a schematic block diagram of apparatus embodying the present invention, and in Figure 12(a) and (b) illustrate frequency characteristics relating to further embodiments of the present invention.

Figures 1 and 2 illustrate basic principles underlying the present invention.

Figure 1 illustrates an example of a reflection type ultrasonic imaging apparatus. In this case, transducers 7 and 8, including electro-acoustic conversion elements, transmit ultrasonic waves A and B to a specimen X. The ultrasonic waves A, B respectively have different frequencies $f_2$, $f_1$.

Ultrasonic wave A is reflected at an area at which there is a change in the nature of internal tissues within the specimen X. That is, reflected waves $C_1$, $C_2$ are returned from boundaries with tissue Y, within specimen X, whilst a reflected wave $C_3$ is returned from the external boundary of specimen X. In the same way, reflected waves $D_1$, $D_2$ and $D_3$ are returned in respect of ultrasonic wave B.

These reflected waves are received by the first and second transducers 7 and 8 and converted into electrical signals. However, the transducers 7 and 8 each receive not only the reflected waves corresponding to their own transmission frequencies but also reflected waves corresponding to the transmission frequency of the other transducer. In other words, each of the transducers 7 and 8 receive both reflected waves $C_1$ to $C_3$ and reflected waves $D_1$ to $D_3$ and then converts them into electrical signals.

At this time, in the case of embodiments of this invention, as will be described later, reflected waves $C_1$ to $C_3$ and reflected waves $D_1$ to $D_3$ are separated and extracted respectively by making use of filtering characteristics provided by transducers 7 and 8. The extracted reflected waves, in time series, are used as tomographic data related to the location of transducers 7 and 8.

In the case of reflection type imaging apparatus, the transducers 7 and 8 may alternatively be used for transmission only, instead of being used in common for transmission and reception. In this case, an electro-acoustic conversion element for reception only is provided.

For B mode operation (see above), the so-called mechanical linear scanning method, wherein the transducers 7 and 8 are mechanically shifted, or the so-called electronic scanning method, wherein many transducers 7 and 8 are arranged in the form of an array and scanning is performed electrically, can be employed as desired. In addition, the so-called sector scanning method can also be introduced.

Figure 2 shows an example of transmission type imaging apparatus, in which the members of electroacoustic conversion element pairs 7' and 9 and 8' and 10 are provided face to face with one another, respectively.

The first and second electro-acoustic conversion elements 7' and 8' transmit ultrasonic waves A and B to the specimen X. The ultrasonic waves A and B have respective different frequencies.

A transmitted wave E obtained after the ultrasonic wave A has passed through the specimen X, including tissue Y, is received by first electro-acoustic conversion element 9 and then converted into an electrical signal. In the same way, a transmitted wave F obtained after the ultrasonic wave B has passed through the specimen X, including tissue Y is received by the second electro-acoustic conversion element 10 and converted into an electrical signal. The electro-acoustic conversion elements 9 and 10 each receive both transmitted waves E and F, as in the case of reflection type apparatus.

In the case of an embodiment of this invention, the transmitted waves E and F are separated and extracted making use of the band-pass characteristics of the elements 9 and 10 themselves.

In the case of transmission type apparatus, it is possible to use only one among the electro-acoustic conversion elements 9 and 10 for reception.

As in the case of reflection type apparatus, a tomographic image can be obtained by mechanical or electronic linear scanning, and sector scanning can also be introduced.

Figure 3 is a time chart, related to Figure 1, further explanatory of the present invention. In Figure 3, transmitted waves and reflected waves corresponding to those described with reference to Figure 1 are indicated on a time axis t. In the interest of clarity the reflected waves C and D are shown separated in time.

Transducer 7 transmits an ultrasonic wave A1, of frequency 2 MHz for example, as shown in Figure 3(a), whilst the transducer 8 transmits an ultrasonic wave B1, of frequency 1 MHz for example, as shown in Figure 3(c). Reflected waves C11, C21, C31 (corresponding to C1, C2, C3 in Figure 1) can be obtained in respect of ultrasonic wave A1, whilst reflected waves D11, D21, D31 (corresponding to D1, D2, D3 in Figure 1) can be obtained in respect of ultrasonic wave B1. Waves received by transducers 7 and 8 are indicated as combined reflected waves in Figure 3, (a) and (c). It will be understood that in Figures 3(a) and (c) reflected waves received by the relevant transducer (7, 8) and of the same frequency as waves (A1, B1) transmitted by the transducer are shown in solid lines; waves received by the relevant transducer of the frequency transmitted by the other transducer are shown in broken lines.

The received waves of frequencies corresponding to respective transmitted ultrasonic waves are separated and extracted from the combined ultrasonic wave signals by the transducers, which, in an embodiment of the present invention, have the same frequency characteristics as band-pass-filters for the frequencies (1 MHz and 2 MHz) corresponding to the ultrasonic waves.

Figure 4 shows, in principle, the frequency characteristics (frequency vs. amplitude characteristics) of ultrasonic wave transducers as used in the present invention.

In Figure 4, frequency f is represented along the horizontal axis, whilst amplitude level (dB) is represented along the vertical axis.

In principle, for a piezoelectric material used for an ultrasonic wave transducer, such as PZT, PVF etc., the frequency characteristic, namely the transmission characteristic applicable in a case in which an electrical signal is converted into an ultrasonic wave signal at the frequency near the resonance frequency $f_0$ of the transducer is as indicated by curve $T_0$ in Figure 4, whilst the frequency characteristic, namely the reception characteristic applicable in a case in which an ultrasonic wave signal is converted into an electrical signal, is as indicated by curve $R_0$.

Therefore, when a piezoelectric element is used as a transducer in ultrasonic imaging apparatus as explained above, for example, when transmission and reception of ultrasonic waves is effected using only one transducer, such a transducer is equivalent to a filter having a band-pass FB. In addition, even when a transducer for transmission only, and a transducer for reception only are individually provided, these transducers together provide an effect which provides a signal pass-band FB, because the transmission and reception characteristics of the respective transducers are combined overall.

Focusing on this point, embodiments of the present invention provide for the discrimination of different kinds (frequencies) of ultrasonic waves making use of the transmission/reception characteristics of such transducers.

Figure 5 illustrates the frequency characteristics of two transducers driven by respective different centre frequencies.

In Figure 5, the broken line curves R1, R2, R2′ represent reception characteristics, namely the acousto-electric conversion frequency characteristics; the chain line curves T1, T2, T2′ represent transmission characteristics, namely the electro-acoustic conversion frequency characteristics.

Moreover, curves R1, T1 are frequency characteristics of a second transducer (8 in Figure 1, 8′ in Figure 2 for transmission 10 in Figure 2 for reception) driven by a centre frequency $f_1$, the curves R2, T2 are frequency characteristics of a first transducer (7 in Figure 1, 7′ in Figure 2 for transmission, 9 in Figure 2 for reception) driven by a centre frequency $f_2$ ($f_2$ is higher than $f_1$), and curves R2′ and T2′′ are frequency characteristics of a first transducer driven by a centre frequency $f_2'$.

In Figure 5, the second transducer (8 in Figure 1, 10 in Figure 2) which receives an ultrasonic wave signal with centre frequency $f_1$ does not receive an ultrasonic wave signal of frequency $f_2$ transmitted from the first transducer (7 in Figure 1, 7′ in Figure 2—see T2 in Figure 5) because of its reception characteristic R1. Moreover, the first transducer (7 in Figure 1, 9 in Figure 2) which receives the ultrasonic wave signal of the centre frequency $f_2$ does not substantially receive the ultrasonic wave signal (T1) of centre frequency $f_1$ because of its reception characteristics R2.

Therefore, in this case, a filter is not required in addition to the transducer(s) and moreover signals in respective frequency bands can be discriminated as outputs.

On the other hand, when obtaining a tomographic image of the abdomen of a human body, for example, it is undesirable to keep the frequency $f_1$ at too low a value, because this would detrimentally effect the time resolution obtainable, and the frequency $f_2$ cannot be set too high because attenuation coefficient is proportional to frequency. In such a case it is also undesirable to increase the difference or provide a large difference between the frequencies $f_1$ and $f_2$. Therefore, frequencies related as indicated by $f_1$, $f_2'$ shown in Figure 5 may be used. As a result, the first reception element (7 in Figure 1, 9 in Figure 2), having reception characteristic R2′, can generate crosstalk wherein a signal containing a signal component CT transmitted from the second transmission (transducer) element (8 in Figure 1, 8′ in Figure 2) is output as an electrical signal. In order to suppress the crosstalk (CT), an electrical filter is provided in either one of or both of the transmission means connected to the transmission element and the reception means connected to the reception element.

Figure 6 illustrates frequency characteristic in a case in which an electrical filter (a high pass filter) is connected to one reception element (for example 7 in Figure 1, 7′ in Figure 2) used in the present invention.

An electrical filter is connected to the first reception element (e.g. 7 in Figure 1, 7′ in Figure 2) and the characteristic indicated by the line LF of the electrical filter is added to the reception characteristic R2′ of the first reception element.

Alternatively, in another example of an embodiment of this invention, a filter having a characteristic such as to reduce the signal component of transmission characteristic T1 which can be received by the reception characteristic R2′ may be added to the transmission means side of the second transmission element (e.g. 8 in Figure 1, 8′ in Figure 2).

An embodiment of the present invention will now be explained in more detail below.

In the following explanation of one embodiment of this invention, reflected wave apparatus, using reflected waves as received waves, is taken as an example, but it will be understood that it is also possible to use transmitted waves or diffracted waves as received waves in embodiments of this invention.

Figure 11 is a block diagram of an embodiment of the present invention. In Figure 11, 1 is a transmission unit; 2 is a reception gate unit; 3 is a reception processing unit; 4 is a display control unit; 5 is a display unit; 6 is a control unit; 7 is the first transducer unit; and 8 is the second transducer unit.

The first transducer unit 7 is composed of five transducers 7a to 7e, whilst the second transducer unit 8 is also composed of five transducers 8a to 8e. The transducer units are well known array type electro-acoustic conversion elements.

Each array type electro-acoustic conversion element comprises a piezoelectric element which transmits ultrasonic waves in accordance with given transmission frequency signals and also generates electrical signals in accordance with reflected waves received from a specimen X.

The transducer 7a to 7e have transmission and reception frequency characteristics T2', R2' as shown in Figure 5, whilst the transducers 8a to 8e have transmission and reception frequency characteristics T1, R1 as shown in Figure 5.

The transmission unit 1 comprises first and second transmission selectors 11 and 12, the first transmission selector 11 corresponds to the first transducer unit 7, whilst the second transmission selector 12 corresponds to the second transducer unit 8.

Each of the transmission selectors 11 and 12 has one input terminal and five output terminals, and the five output terminals of the first transmission selector 11 are connected to respective transducers 7a to 7e of the first transducer unit 7. The five output terminals of the second transmission selector 12 are connected to respective transducers 8a to 8e of the second transducer unit 8. The input terminals of the transmission selectors 11 and 12 are respectively connected to first and second signal generator circuits (transmission circuits) 13 and 14. The input terminal and the output terminals of a transmission selector (11 and 12) are connected by means of a rotary arm, which sequentially and selectively connects the input terminal to each output terminal in accordance with instruction sent from a selection circuit 15.

The transmission selectors 11 and 12 have been explained in terms of a rotary arm and contacts for ease of understanding. However, it is desirable to use a gate circuit structure for a transmission selector, for example a well known select circuit.

The selection circuit 15 changes the connections of the transmission selectors 11 and 12 in accordance with an instruction from the control unit 6, and the first and second signal generator circuits 13 and 14 output signals for generating ultrasonic waves A and B (see Figure 1) upon receipt of instruction from the control unit 6.

Therefore, in the transmission unit 1, the first signal generator circuit 13 outputs a signal for causing transmission of the ultrasonic wave A of frequency $f_2'$, whilst the second signal generator circuit 14 outputs a signal for causing transmission of the ultrasonic wave B of frequency $f_1$, upon receipt of instruction from the control unit 6, and these signals are sent to (selected) transducers 7a to 7e and 8a to 8e connected to respective selection output terminals of the transmission selectors 11 and 12, in accordance with instruction sent from the selection circuit 15.

Transducers 7a and 8a are first selected, and the ultrasonic wave A is transmitted from transducer 7a whilst the ultrasonic wave B is transmitted from the transducer 8a, simultaneously. In conformity with an instruction sent from the selection circuit 15, the transmission selectors 11 and 12 then select respective second output terminals thereof, and therefore the first and second signal generator circuits 13 and 14 are respectively connected to the transducers 7b and 8b. Then, when an instruction is received from the control unit 6, output signals corresponding to the ultrasonic waves A and B are generated from the first and second signal generator circuits 13 and 14 and thereby ultrasonic waves A and B are transmitted from the transducers 7b and 8b, and so on.

Here, so-called electronic linear scanning is performed simultaneously by two transducers.

The transmission time chart of the transducers 7 and 8 at respective frequencies is as shown in Figure 3.

The transmission period or interval from transmission by transducer 7a to transmission by transducer 7b is the same as the transmission interval of existing methods.

The first and second signal generator circuits 13 and 14, supply pulsed output signals. This is not explained in detail because well known circuit structures are employed.

The reflected waves resulting from ultrasonic waves which are thus transmitted by the transmission unit 1 are processed by the reception gate unit 2 and reception processing unit 3.

A first reception circuit comprises a first reception selector 21, a first gain compensating circuit 23, an extraction filter 31 and a first shift register 32. A second reception circuit comprises a second reception selector 22, a second gain compensating circuit 24 and a second shift register 33.

The reception gate unit 2 provides the first and second reception selectors 21 and 22, and the first reception selector 21 corresponds to the first transducer unit 7, whilst the second reception selector 22 corresponds to the second transducer unit 8.

The reception selectors 21 and 22 each have one output terminal and five input terminals. The five input terminals of the first reception selector 21 are connected to respective transducers 7a to 7e of the first transducer unit 7. The five input terminals of the second reception selector 22 are connected to respective transducers 8a to 8e of the second transducer unit 8. The output terminals of the reception selectors 21 and 22 are respectively connected to the first and second gain compensating circuits 23 and 24.

The output terminal of a reception selector (21 and 22) is connected with the input terminals of the selector by means of a rotary arm. The rotary arm sequentially and selectively connects the output terminal to each input terminal in accordance with instruction given from a selection circuit 25.

The reception selectors 21 and 22 are explained in terms of rotary arm and contacts for ease of understanding. However, it is desirable to use a gate circuit structure, for example, the well known select circuit.

The selection circuit 25 changes the connections of the reception selectors 21 and 22 according to instruction sent from the control unit 6.

The gain compensating circuits 23, 24 compensate for attenuation, by changing the gain on a time basis (e.g. increasing gain as time after transmission increases), because a reflected wave returning from a further or deeper area in a specimen, which takes longer to return, suffers greater attenuation. The gain compensating circuits 23 and 24 change gain on a time basis in response to instruction generated by the control unit 6 after it has generated an instruction for the output of ultrasonic waves.

The reception gate unit 2 connects the transducers 7a to 7e, 8a to 8e, connected to input terminals of the reception selectors 21 and 22, to the output terminals of selectors 21 and 22 according to instructions sent from the selection circuit 25. The electrical signals obtained from the reflected waves sent from the transducers 7a to 7e, 8a to 8e are respectively input to the gain compensating circuits 23 and 24.

In Figure 11, the transducers 7a, 8a are first selected and an electrical signal corresponding to the reflected wave received by the transducer 7a is input to the gain compensating circuit 23, whilst an electrical signal corresponding to the reflected wave received by the transducer 8a is input to the gain compensating circuit 24.

The reception selectors 21 and 22 then sequentially select, like the transmission selectors 11 and 12, the transducers 7b and 8b, 7c and 8c, 7d and 8d, 7e and 8e, for electronic scanning in synchronization with the selection operation of the transmission selectors.

C11 to C31, D11 to D31 in Figure 3 respectively show the received ultrasonic waves of the transducers 7a and 8a.

The outputs of the gain compensating circuits 23 and 24 are input to the reception processing unit 3.

The reception processing unit 3 comprises an extraction filter 31 having characteristics as shown by LF in Figure 6, and the first and second shift registers 32, 33. The extraction filter 31 passes only the reflected wave (frequencies) corresponding to the transmitted ultrasonic wave A, namely the ultrasonic wave of centre frequency $f_2'$ as shown in Figure 6, and then sends it to the shift register 32 after analog-to-digital conversion. On the other hand, as explained in Figure 5, the output signal of transducer 8 contains only the reflected waves corresponding to the ultrasonic wave B, namely the ultrasonic wave of centre frequency $f_1$, and then sends it to the shift register 33 after analog-to-digital conversion.

Therefore, only those reflected waves corresponding to its own transmission frequencies are extracted from among all the reflected waves received by the transducer unit 7, as a result of the filter function provided by reception characteristic R2′ (Figure 6) of the transducers 7a to 7e and the filter characteristic LF (Figure 6) of the extraction filter 31. On the other hand, only those reflected waves corresponding to its own transmission frequencies are extracted from amongst all the reflected waves received by the transducer unit 8 as a result solely of the filter function provided by the reception characteristic R1 (Figure 5) of the transducers 8a to 8e.

The data thus stored in the shift registers 32, 33 is digital values representing a time series of reflected waves corresponding to the transmitted ultrasonic waves.

The time series digital values are input to the display control unit 4 for display on the display unit 5.

The display control unit 4 comprises a screen memory 41, a write circuit 40 and a display control circuit 42. The write circuit 40 writes output signals from the two shift registers 32 and 33 into corresponding locations of the screen memory 41, according to the location of the transducers 7a to 7e, 8a to 8e.

The display control circuit 42 transmits the stored information from the screen memory 41, according to the scanning timing of the display unit 5, causing the unit 5 to display a tomographic image on the basis of the time series reflected wave data.

The ultrasonic waves A and B are transmitted, resultant reflected waves are received and a tomographic image is displayed on the display unit through separation and extraction.

In the above embodiment of this invention a high-pass filtering function is provided for the one receiving signal on the reception side, but other methods may be employed in accordance with embodiments of this invention.

Figure 12(a) and (b) illustrate frequency characteristics relating to further embodiments of the present invention. In these Figures, items equivalent to those seen in Figure 5 are given the same reference symbols. Namely, in the case of Figure 12(a), a filter (low-pass) having a filtering characteristic LF′ is inserted between the second signal circuit 14 and the transducer 8 of the transmission unit 1 in Figure 11. In this case, the filter 31 in Figure 11 can be removed, but if it is retained, crosstalk can be eliminated perfectly. (See also description of Figure 7 below).

In the case of Figure 12(a), the transmission spectrum SA1 exists at least in the band which is not extracted by the reception characteristic R2 of the transducer 7.

Figure 12(b) illustrates frequency characteristics for a case in which three transducer units are used. The bands of two transmitting signals are defined by hatched areas SA1 and SA2 to which the signals are limited by means of filter characteristics LF1 (low-pass), LF2 (band-pass), and these signals are received selectively by limiting the signal to the band SA3 by the characteristic of the transducer.

Therefore, even when filters are used in transmission or reception circuits, the number of filters required can be reduced as long as the filtering characteristic of a transducer is utilized for the discrimination of signals.

Figure 7 illustrates frequency characteristics for an example in which electrical filters are connected to both the first and second reception elements in an embodiment of the present invention.

In the examples of Figure 6 and Figure 11, an electrical (high-pass) filter is provided in relation to the first reception elements in order to cut the reception of signals related to frequency $f_1$ by the first reception elements. When the frequency spacing between transducers becomes narrow, as shown in Figure 7, the characteristic T2 of the first transmission element and the characteristic R1 of the second reception element overlap (contrast Figure 5), thus causing crosstalk.

Such crosstalk is prevented by providing an electrical filter having the characteristic indicated by F1 in Figure 7 in the reception circuit of the second reception element.

It is also possible, additionally, or alternatively, to provide an electrical filter having the characteristic indicated by F2 in Figure 7 in relation to the transmission element.

Moreover, it is more desirable, in some cases, to replace such a (high- or low-pass) electrical filter with a band-pass filter.

Figure 8 illustrates frequency characteristics in a case in which a band-pass filter is used in an embodiment of the present invention.

Namely, a second electrical filter, having the characteristic F1 as seen in Figure 8, is connected to the second reception element, whilst a first filter having the characteristic F2 seen in Figure 8, is connected to the first reception element.

Thereby, the efficiency of crosstalk elimination can be much improved.

For example, the centre frequency of a transducer can be determined in dependence upon the thickness of piezoelectric material used. Therefore, as shown in Figure 8, for example, the transmission spectrum of the second transmission element is accompanied by harmonics T1' ($3f_1$) in addition to T1. At this time, the component T1' enters the reception signal band of the first reception element. Such a component can be eliminated by using a band-pass filter in place of the first (high-pass) electrical filter. It is also recommended to use a band-pass filter in place of the second (low-pass) electrical filter in order to efficiently eliminate unwanted noise.

The crosstalk elimination effect becomes perfect when electrical filters are connected to all of the first and second transmission elements and the first and second reception elements.

Attenuation is greater at higher frequencies than at lower frequencies. A method of compensating for such attenuation will be explained below.

Figure 9 illustrates a frequency characteristic in a case in which steps are taken for reducing the effects of attenuation in an embodiment of the present invention.

The transmission level of the first transmission element is raised as shown by T2C, above that shown in Figure 7. This purpose is attained by raising the transmission level of the signal generator circuit 13 shown in Figure 11. Thereby, the high frequency side $f_1$ is higher in level than the low frequency side $f_2$, compensating for attenuation of the higher frequency component.

Figures 10A and 10B illustrate the characteristics of electrical filters used in embodiments of the present invention.

In Figure 10A, an electrical filter having the characteristic F2 is connected to the first reception element, whilst an electrical filter having the characteristic F1 is connected to the second reception element. An electrical filter is so designed that the characteristic is flat up to a frequency corresponding to −3dB of the transmission, reception characteristics R1, T2 but then has a predetermined slope at the frequency corresponding to the level lower than −3dB.

This characteristic assures a wide band and a sufficient cut-off function of the filter.

Figure 10B shows the characteristic obtained by connecting an electrical filter having a characteristic F2' to the first reception element and an electrical filter having the characteristic F1' to the second transmission element.

In this case, an electrical filter is so designed that the characteristic is flat up to the frequency corresponding to −3dB of the reception, transmission characteristics T1, R2, but shows a predetermined slope as in the case of Figure 10A, at the frequency corresponding to the level lower than −3dB.

On the other hand, the overall filtering effect as a system is indicated on the basis of multiplication: therefore, the transmission characteristic becomes F1'×T1, whilst the reception characteristic becomes F2'×R2. When a filter provided as shown in Figure 10A or 10B is adopted, a characteristic assuring a wide frequency band and little crosstalk can be obtained.

In above embodiments of this invention, impulse signals are used as the ultrasonic waves transmitted, but various other kinds of signals such as burst wave signals, chirp modulated wave signals etc., can also be used.

As explained previously, signal components in different frequency bands of the ultrasonic wave band can be separated and output according to the present invention. Therefore, a plurality of specimen data can be obtained simultaneously. This is very effective in relation to diagnostic operation in medicine or in industry.

Thus, an embodiment of this invention can provide an improvement in ultrasonic imaging apparatus having a transducer for transmitting ultrasonic waves and receiving acoustic waves and in which ultrasonic waves of different frequencies are transmitted simultaneously from a plurality of transducers.

In an embodiment of the present invention, the frequency characteristics of transducers are determined in such a manner as to provide the function of a frequency filter, and moreover an electrical filter is additionally provided for compensation. Thereby crosstalk can be eliminated even when frequency difference between different frequency transmitted ultrasonic waves is small.

**Claims**

1. Ultrasonic imaging apparatus, for imaging the internal structure of an object (X) by transmitting an ultrasonic wave (A, B; A1, B1, A1', B1') into the object (X) and receiving an acoustic wave (C1 to C3, D1 to D3; C11 to C31, D11 to D31; E, F) from the object (X), the apparatus including at least two transmit/receive transducer means (7, 8; 7', 9, 8', 10; 7a to 7e; 8a to 8e), for transmitting ultrasonic waves and receiving acoustic waves, having respective different frequency characteristics and functioning as frequency filters by virtue of their own frequency characteristics,

each of said transducer means having a transmission characteristic (T0; T1, T2, T2'; T2C) and a reception characteristic (R0; R1, R2, R2') both including a transducer resonance frequency (F0, F1, f2, f2'), the transmission characteristic extending above the resonance frequency beyond the reception characteristic and the reception characteristic extending below the resonance frequency beyond the transmission characteristic,

a first transmission circuit (14, 12) connected to a first (8, 8a, to 8e; 8', 10) of the said transmit/receive transducer means, for causing it to transmit an ultrasonic wave of a first frequency (f1),

a first reception circuit (22, 24, 33) connected to the first transmit/receive transducer means,

a second transmission circuit (13, 11), connected to a second (7, 7a, to 7e; 7', 9) of the said transmit/receive transducer means, for causing it to transmit an ultrasonic wave of a second frequency (f2, f2'), higher than the first frequency (f1),

a second reception circuit (21, 23, 31, 32) connected to the second transmit/receive transducer means, and

an electrical filter (31) provided in the first transmission circuit or in the second reception circuit, the filter characteristic (LF, F2, LF'; LF1) of the electrical filter being determined in dependence upon the transmission characteristic (T1) of the first transmit/receive transducer means and the reception characteristic (R2, R2') of the second transmit/receive transducer means, so as to reduce crosstalk between the transducer means.

2. Apparatus as claimed in claim 1, wherein the reception characteristic (R1) of the first transmit/receive transducer means (8, 8a to 8e; 8', 10) partly overlaps the transmission characteristic (T2, T2', T2C) of the second transmit/receive transducer means, (7, 7a to 7e; 7', 9) and the electrical filter (31) has the function of eliminating such overlap.

3. Apparatus as claimed in claim 1 or 2, wherein respective electrical filters are provided in both the first transmission circuit and the second reception circuit.

4. Apparatus as claimed in claim 1, 2 or 3, wherein an additional electrical filter is provided in the second transmission circuit or the first reception circuit, the characteristic (F1, LF2) of the additional electrical filter being determined in dependence upon the transmission characteristic of the second transmit/receive transducer means and the reception characteristic of the first transmit/receive transducer means, so as to reduce further crosstalk between the tranducer means.

5. Apparatus as claimed in claim 4, wherein respective additional electrical filters are provided in both the second transmission circuit and the first reception circuit.

6. Apparatus as claimed in any preceding claim, wherein the first and second transmit/receive transducer means comprise respective transducers (8, 8a to 8e; 7, 7a to 7e) operable to transmit ultrasonic waves and to receive acoustic waves.

7. Apparatus as claimed in any one of claims 1 to 5, wherein at least one of the first and second transmit/receive transducer means comprises respective transducers for transmission only (7', 8') and for reception (9, 10) only.

**Patentansprüche**

1. Ultraschall-Abbildungsvorrichtung, zur Abbildung der inneren Struktur eines Objektes (X) durch Sendung einer Ultraschallwelle (A, B; A1, B1, A1', B1') in das Objekt (X) und Empfangen einer akustischen Welle (C1 bis C3, D1 bis D3; C11 bis C31, D11 bis D31; E, F) von dem Objekt (X), wobei die Vorrichtung umfaßt: wenigstens zwei Sende/Empfangs-Wandlereinrichtungen (7, 8; 7', 9, 8', 10; 7a bis 7e; 8a bis 8e), zur Sendung von Ultraschallwellen und zum Empfang von akustischen Wellen, die jeweils verschiedene Frequenzcharakteristiken haben und, aufgrund ihrer eigenen Frequenzcharakteristiken, als Frequenzfilter wirken,

wobei jede der genannten Wandlereinrichtungen eine Sendecharakteristik (T0; T1, T2, T2'; T2C) und eine Empfangscharakteristik (R0; R1, R2, R2') hat, die beide eine Wandlerresonanzfrequenz (f0, f1, f2, f2') einschließen, wobei die Sendecharakteristik sich oberhalb der Resonanzfrequenz jenseits der Empfangscharakteristik erstreckt und die Empfangscharakteristik sich unterhalb der Resonanzfrequenz jenseits der Sendecharakteristik erstreckt,

eine erste Sendeschaltung (14, 12), die mit einer ersten (8, 8a bis 8e; 8', 10) der genannten Sende/Empfangs - Wandlereinrichtungen verbunden ist, um zu bewirken, daß diese eine Untraschallwelle einer ersten Frequenz (f1) sendet,

eine erste Empfangsschaltung (22, 24, 33), die mit der ersten Sende/Empfangs-Wandlereinrichtung verbunden ist,

eine zweite Sendeschaltung (13, 11), die mit einer zweiten (7, 7a bis 7e; 7'; 9) der genannten Sende/Empfangs - Wandlereinrichtungen verbunden ist, um zu bewirken, daß diese eine Ultraschallwelle von einer zweiten Frequenz (f2, f2') sendet, die höher als die erste Frequenz (f1) ist,

eine zweite Empfangsschaltung (21, 23, 31, 32), die mit der zweiten Sende/Empfangs-Wandlereinrichtung verbunden ist, und ein elektrisches Filter (31), das in der ersten Sendeschaltung oder in der zweiten Empfangs-schaltung enthalten ist, wobei die Filtercharakteristik (LF, F2, LF', LF1) des elektrischen Filters in Abhängigkeit von der Sendecharakteristik (T1) der ersten Sende/ Empfangs-Wandlereinrichtung und der Empfangscharakteristik (R2, R2') der zweiten Sende/ Empfangs-Wandlereinrichtung bestimmt wird, um so ein Nebensprechen zwischen den Wandlereinrichtungen zu reduzieren.

2. Vorrichtung nach Anspruch 1, bei welcher die Empfangscharakteristik (R1) der ersten Sende/Empfangs-Wandlereinrichtung (8, 8a bis 8e; 8', 10) teilweise die Sendecharakteristik (T2, T2', T2C) der zweiten Sende/Empfangs-Wandlereinrichtung (7, 7a, bis 7e; 7', 9) überlappt und das elektrische Filter (31) die Funktion hat, solch ein überlappen zu eliminieren.

3. Vorrichtung nach Anspruch 1 oder 2, bei welcher elektrische Filter sowohl in der ersten Sendeschaltung als auch in der zweiten Empfangsschaltung vorgesehen sind.

4. Vorrichtung nach Anspruch 1, 2 oder 3, bei welcher ein zusätzliches elektrisches Filter in der zweiten Sendeschaltung oder der ersten Empfangsschaltung vorgesehen ist, die Charakteristik (F1, LF2) des zusätzlichen elektrischen Filters in Abhängigkeit von der Transmissionscharakteristik der zweiten Sende/Empfangs-Wandlereinrichtung und der Erfolgscharakteristik der ersten Sende/Empfangs-Wandlereinrichtung bestimmt wird, um so ein Nebensprechen zwischen den Wandlereinrichtungen weiter zu reduzieren.

5. Vorrichtung nach Anspruch 4, bei welcher entsprechende zusätzliche elektrischef Filter sowohl in der zweiten Transmissionsschaltung als auch in der ersten Empfangsschaltung vorgesehen sind.

6. Vorrichtung nach einem der vorhergehenden Ansprüche, bei welcher die erste und zweite Sende/Empfangs-Wandlereinrichtung entsprechende Wandler (8, 8a bis 8e; 7, 7a bis 7e) umfaßt, die zur Übertragung von Ultraschallwellen und zum Empfang von akustischen Wellen dienen.

7. Vorrichtung nach einem der Ansprüche 1 bis 5, bei welcher wenigstens eine der ersten und zweiten Sende/Empfangs-Wandlereinrichtungen entsprechende Wandler nur für die Übertragung (7', 8') und nur für den Empfang (9, 10) umfaßt.

**Revendications**

1. Appareil de formation d'images ultrasons, destiné à former l'image de la structure interne d'un objet (X) par émission d'une onde ultrasonore (A, B; A1, B1, A1', B1') dans l'objet (X) et par réception d'une onde acoustique (C1 à C3, D1 à D3; C11 à C31, D11 à D31; E, F) provenant de l'objet (X), l'appareil comportant au moins deux dispositifs transducteurs d'émission/réception (7, 8; 7', 9, 8', 10; 7a à 7e; 8a à 8e) pour émettre des ondes ultrasonores et recevoir des ondes acoustiques, ayant des caractéristiques de fréquences différentes respectives et fonctionnant comme des filtres de fréquence en raison de leurs propres caractéristiques en fréquence, chacun desdits dispositifs transducteurs ayant une caractéristique d'émission (To; T1, T2, T2'; T2C) et une caractéristique de réception (R0, R1, R2, R2') contenant chacune une fréquence de résonance de transducteur (f0, F1, f2, f2'), la caractéristique d'émission se prolongeant au-dessus de la fréquence de résonance au-delà de la caractéristique de réception, et la caractéristique de réception se prolongeant au-dessous de la fréquence de résonance au-delà de la caractéristique d'émission, un premier circuit d'émission (14, 12) connecté à un premier (8, 8a à 8e; 8', 10) desdits dispositifs transducteurs d'émission/réception pour qu'il émette une onde ultrasonore d'une première fréquence (f1), un premier circuit de réception (22, 24, 33) connecté au premier dispositif transducteur d'émission/réception, un second circuit d'émission (13, 11) connecté à un second (7, 7a à 7e; 7', 9) desdits dispositifs transducteurs d'émission/réception pour qu'il émette une onde ultrasonore d'une seconde fréquence (f2, f2') supérieure à la première fréquence (f1), un second circuit de réception (21, 23, 31, 32) connecté au second dispositif transducteur d'émission/réception, et un filtre électrique (31) prévu dans le premier circuit d'émission ou dans le second circuit de réception, la caractéristique de filtrage (LF, F2, LF', LF1) du filtre électrique étant déterminée en fonction de la caractéristique d'émission (T1) du premier dispositif transducteur d'émission/réception et de la caractéristique de réception (R2, R2') du second dispositif transducteur d'émission/réception de manière à réduire la transmodulation entre les dispositifs transducteurs.

2. Appareil selon la revendication 1, dans lequel la caractéristique de réception (R1) du premier dispositif transducteur d'émission/réception (8, 8a à 8e; 8', 10) revouvre partiellement la caractéristique d'émission (T2, T2', T2C) du second dispositif transducteur d'émission/réception (7, 7a à 7e; 7', 9), le filtre électrique (31) ayant pour fonction d'éliminer ce chevauchement.

3. Appareil selon la revendication 1 ou 2, dans lequel des filtres électriques respectifs sont prévus à la fois dans la premier circuit d'émission et dans le second circuit de réception.

4. Appareil selon la revendication 1, 2 ou 3 dans lequel un filtre électrique supplémentaire est prévu dans le second circuit d'émission ou le premier circuit de réception, la caractéristique (F1, LF2) du filtre électrique supplémentaire étant déterminée en fonction de la caractéristique d'émission du second dispositif transducteur d'émission/réception et de la caractéristique de réception du premier dispositif transducteur d'émission/réception de manière à réduire encore la transmodulation entre les dispositifs transducteurs.

5. Appareil selon la revendication 4, dans lequel des filtres électriques supplémentaires respectifs sont prévus à la fois dans le second circuit d'émission et dans le premier circuit de réception.

6. Appareil selon l'une quelconque des revendications précédentes, dans lequel le premier et le second dispositif transducteur d'émission/réception comportent des transducteurs respectifs (8, 8a à 8e; 7, 7a à 7e) ayant pour fonction d'émettre des ondes ultrasonores et de recevoir des ondes acoustiques.

7. Appareil selon l'une quelconque des revendications 1 à 5, dans lequel l'un au moins des premier et second dispositifs transducteurs d'émission/réception comporte des transducteurs respectifs pour l'émission seulement (7', 8') et pour la réception seulement (9, 10).

Fig. 1

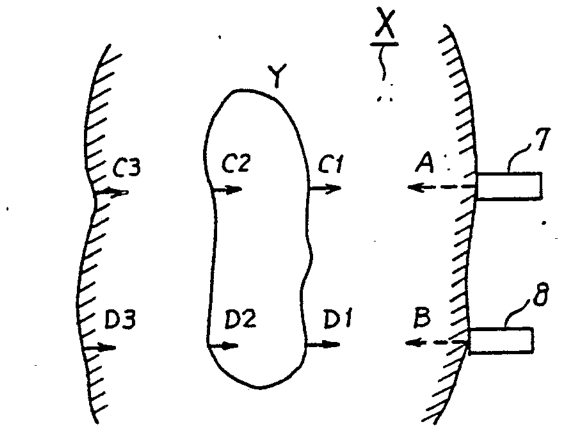


Fig. 2

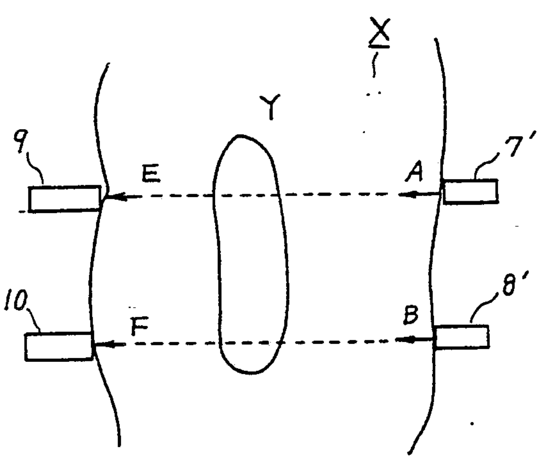

Fig. 3

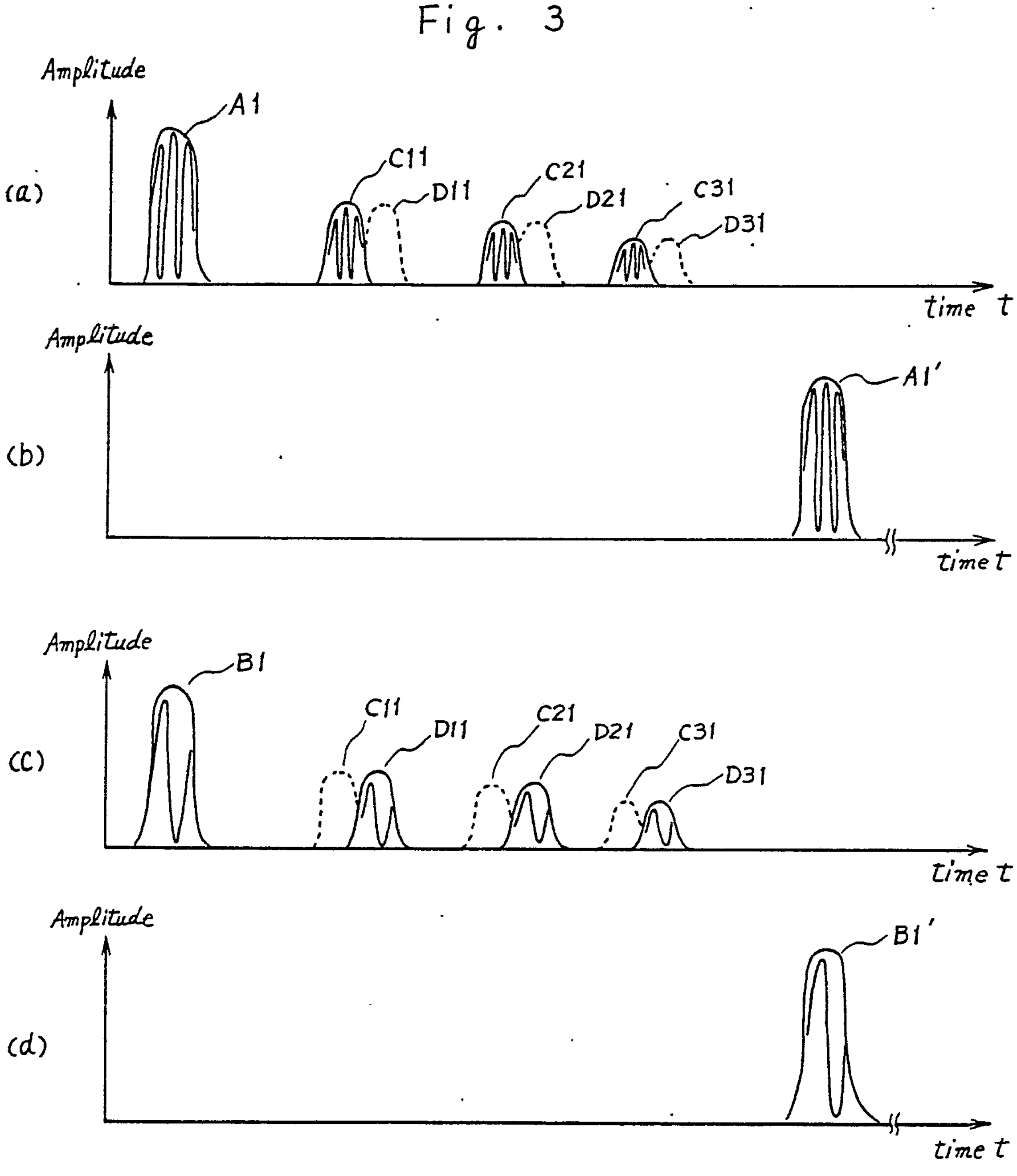
Amplitude
A1
(a)
C11
D11
C21
D21
C31
D31
time t
Amplitude
A1'
(b)
time t
Amplitude
B1
(c)
C11
D11
C21
D21
C31
D31
time t
Amplitude
B1'
(d)
time t

Fig. 4

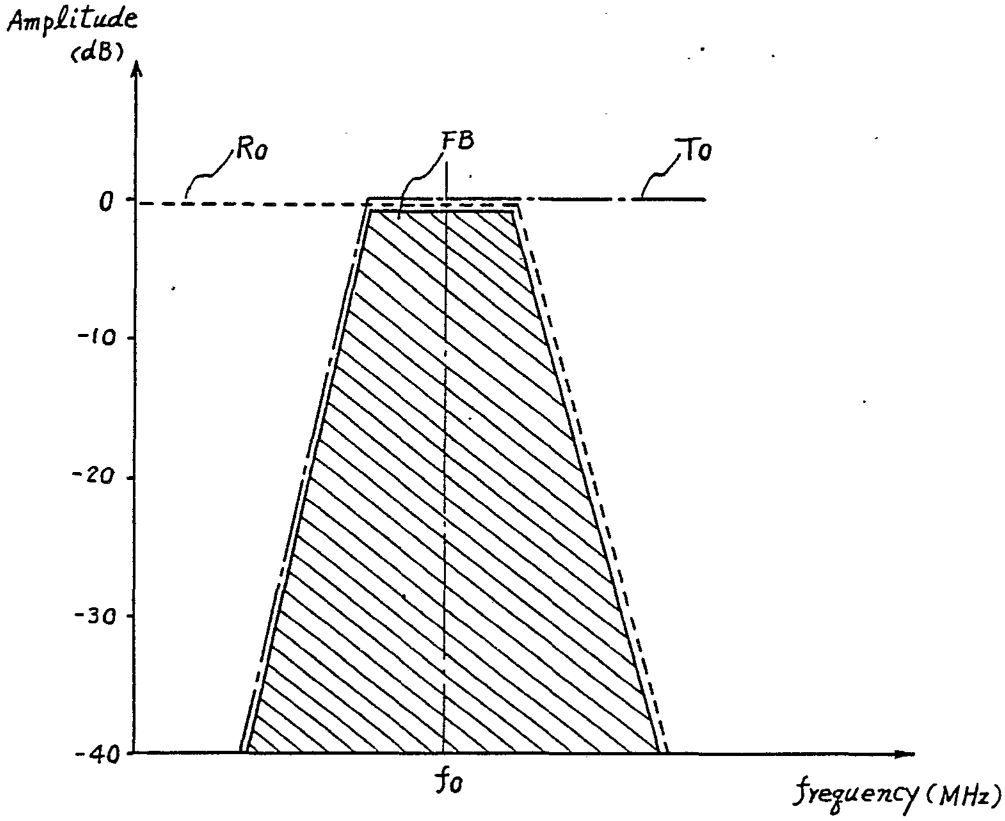

Fig. 5

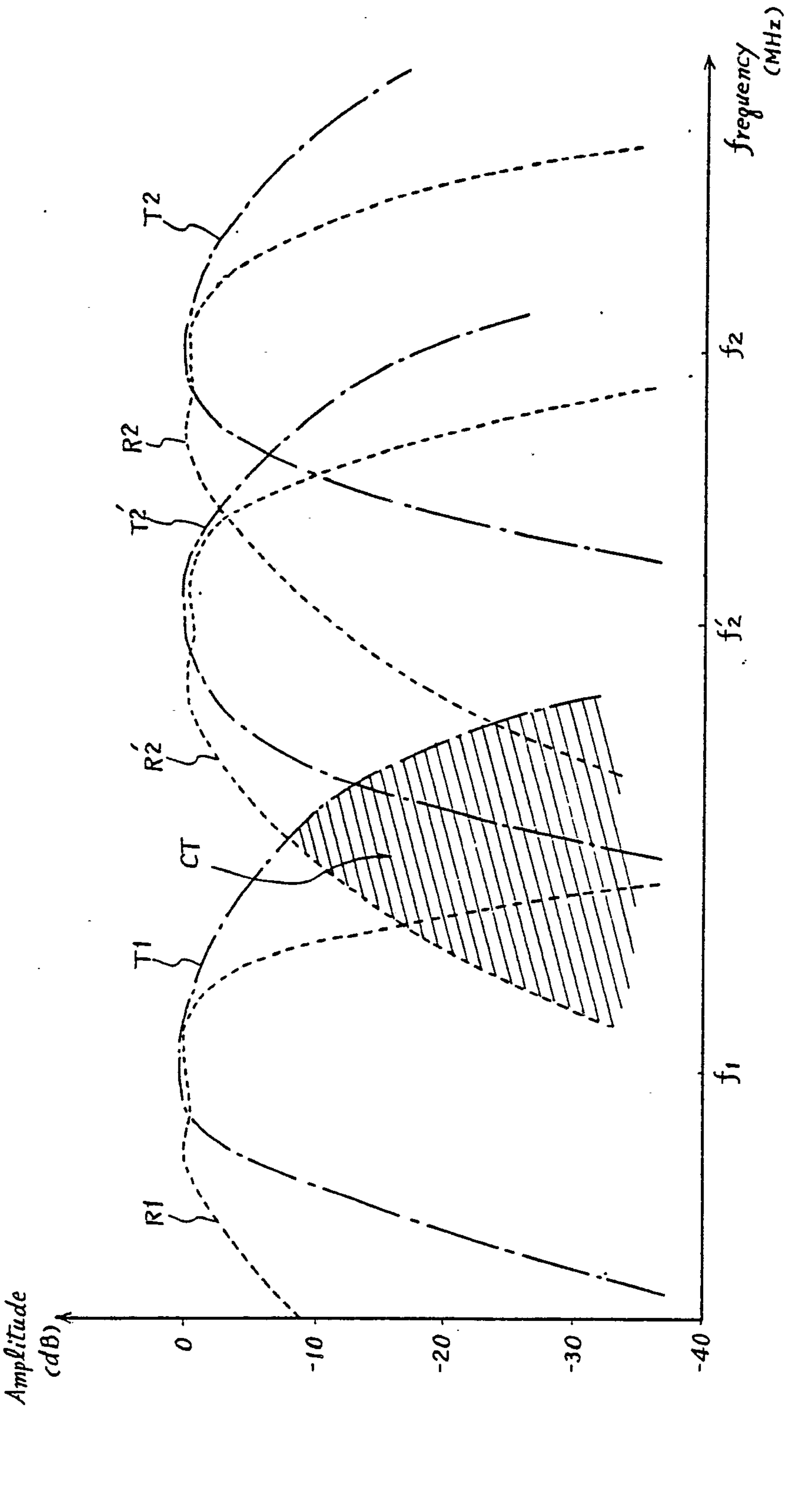
Amplitude (dB)
0
-10
-20
-30
-40
frequency (MHz)
f1
f'2
f2
R1
T1
CT
R'2
T'2
R2
T2

Fig. 6

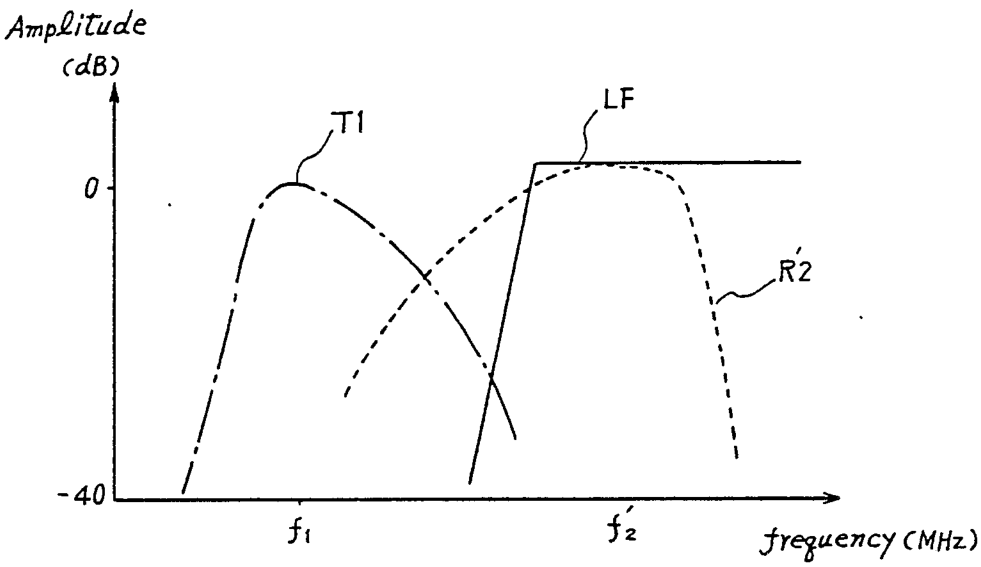

Fig. 7

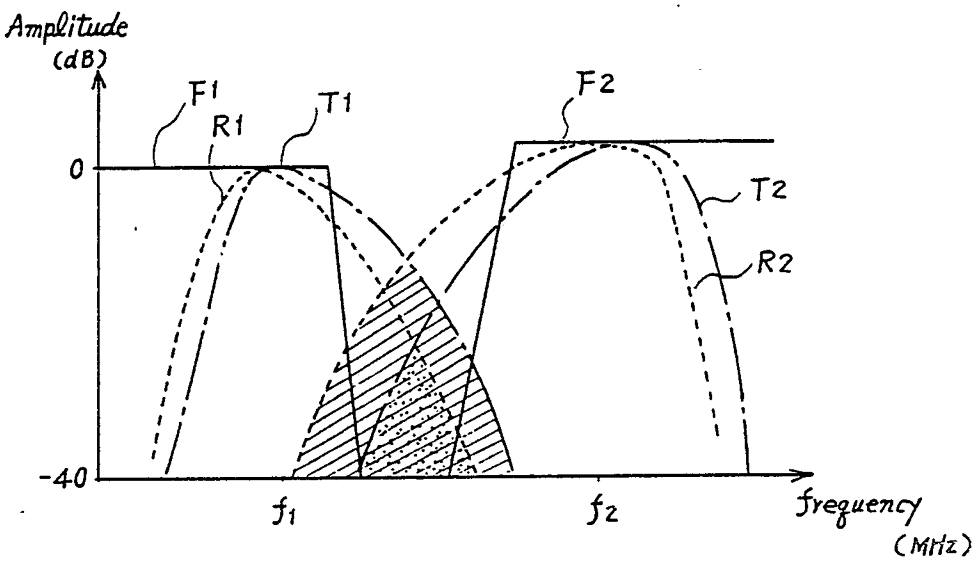

Fig. 8

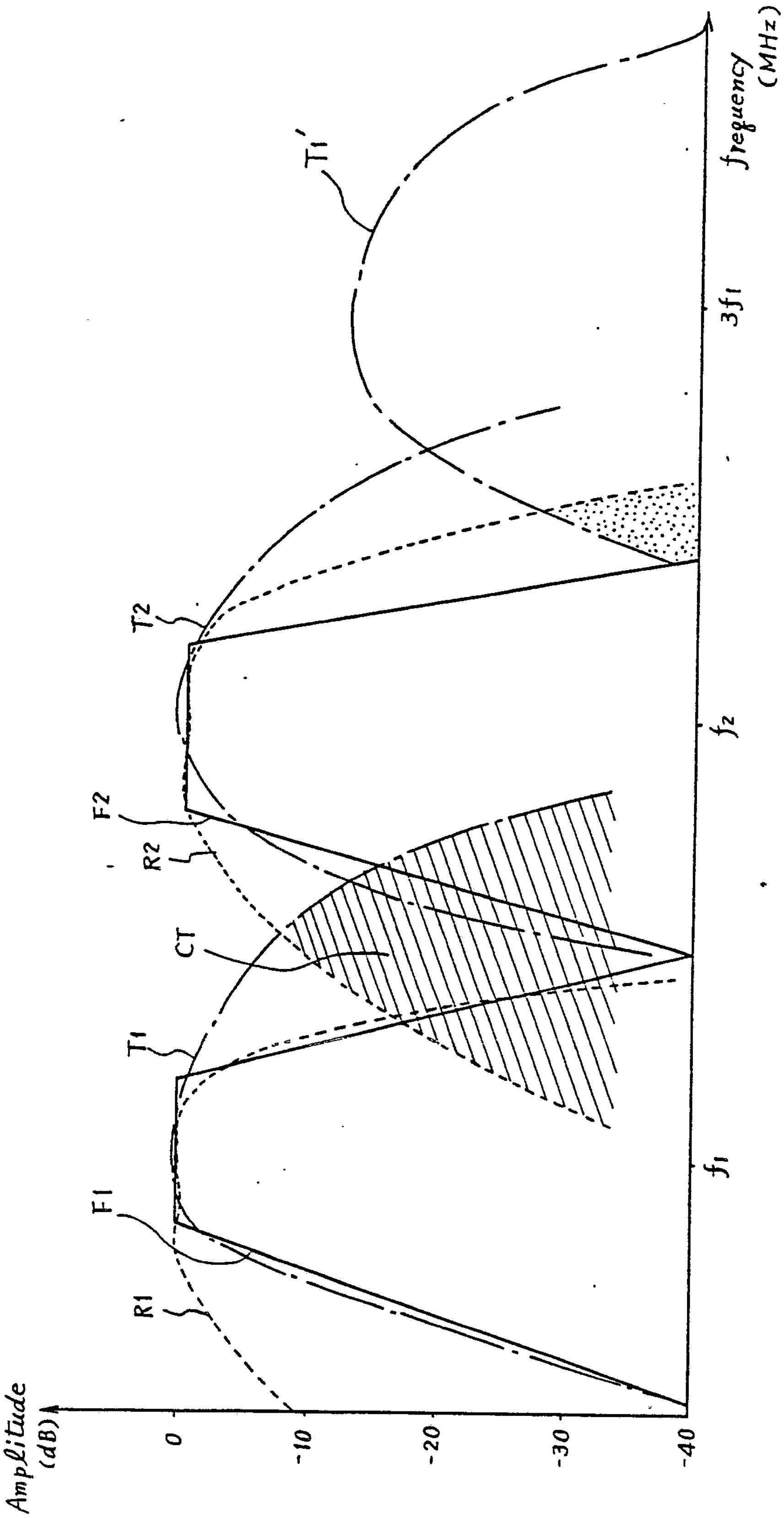
Amplitude
(dB)
0
-10
-20
-30
-40
R1
F1
T1
CT
R2
F2
T2
T1'
f1
f2
3f1
frequency
(MHz)

Fig 9

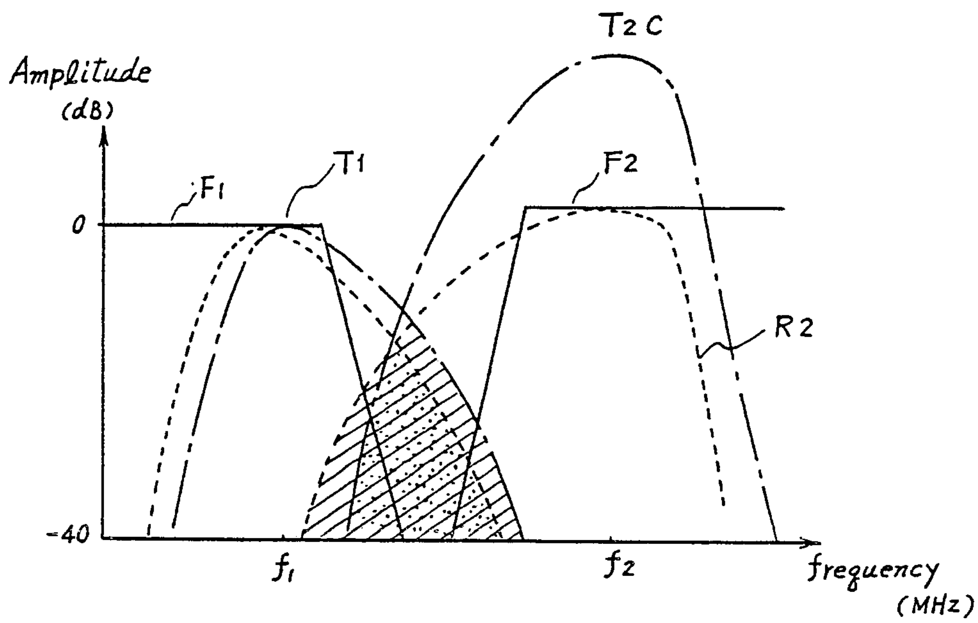

Fig. 10 A

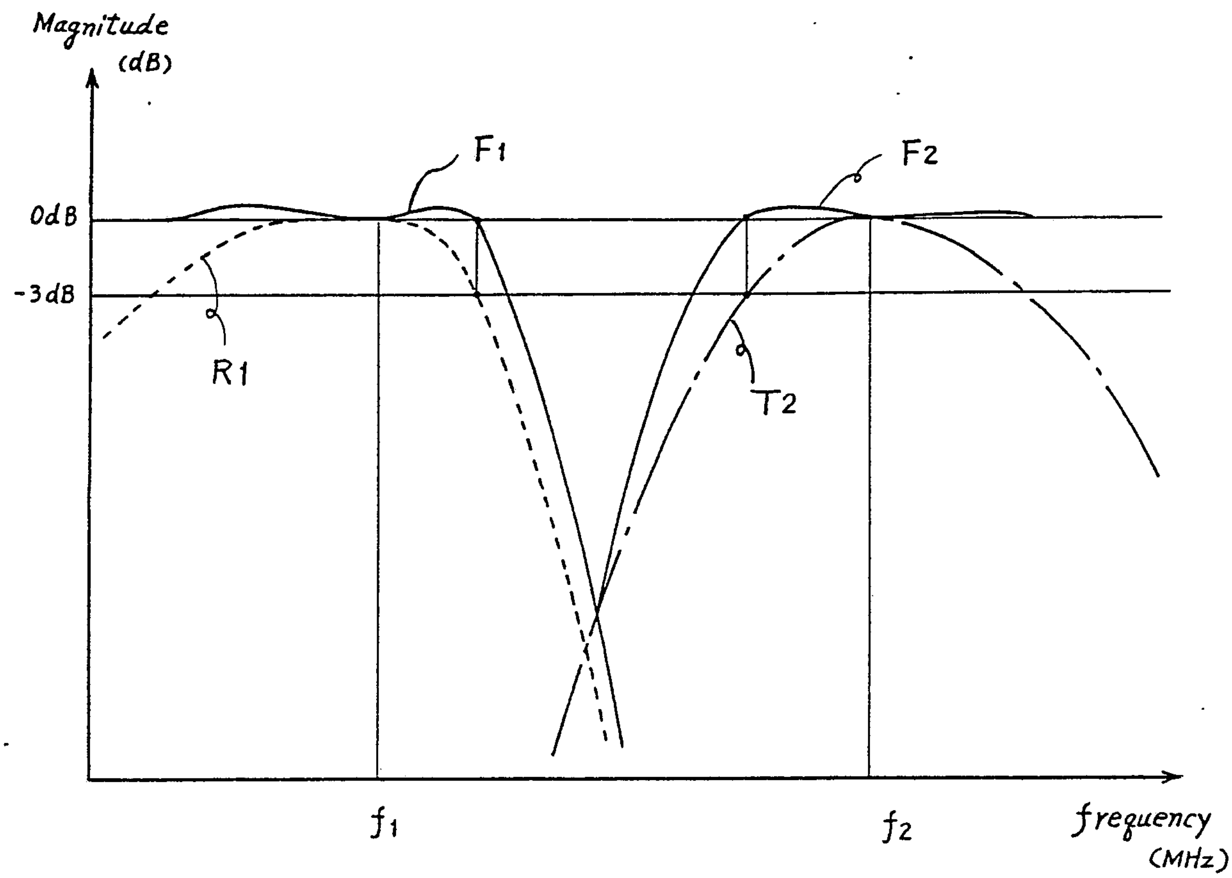

Fig. 10 B

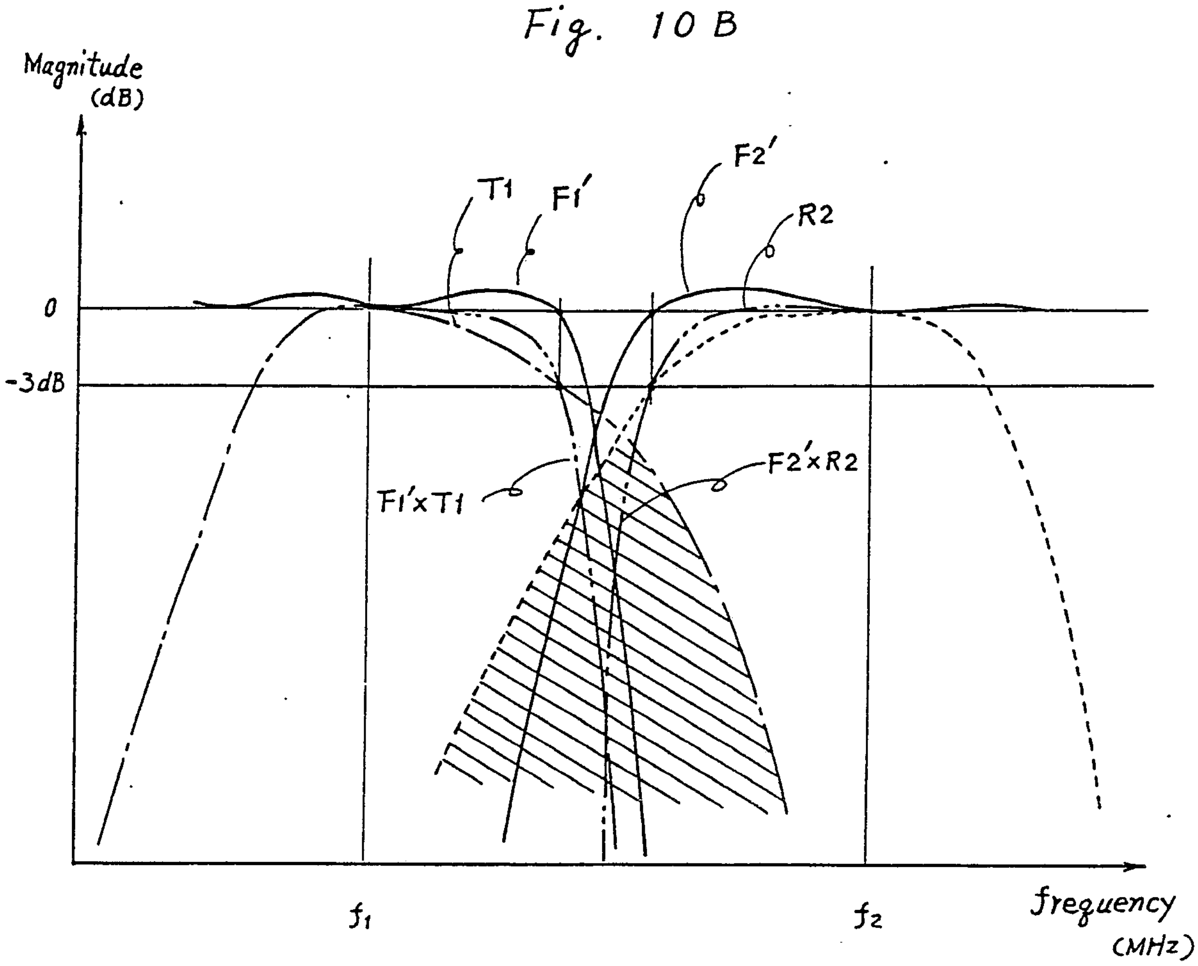

Fig. 11

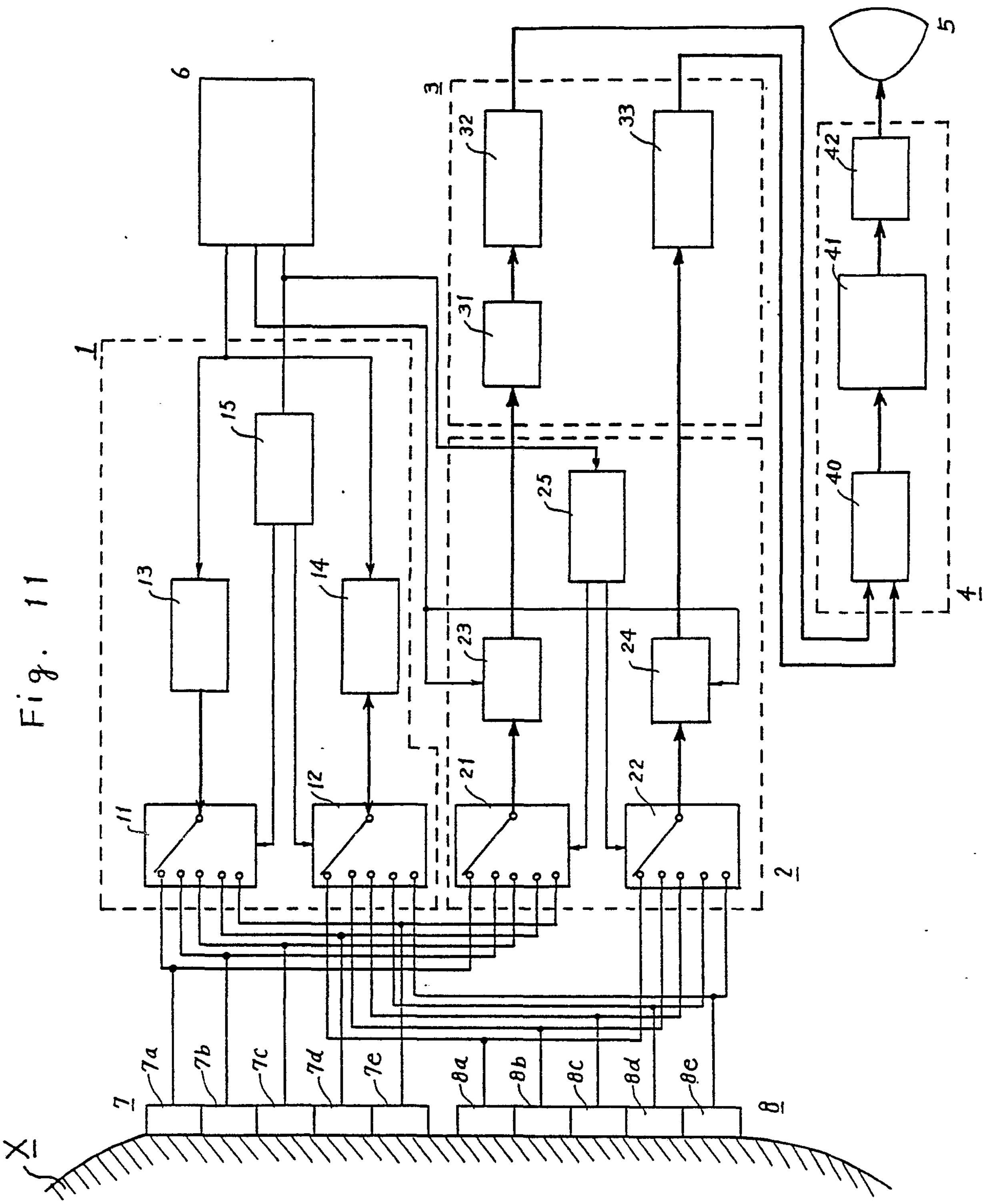
1
2
3
4
5
6
7
8
X
7a
7b
7c
7d
7e
8a
8b
8c
8d
8e
11
12
13
14
15
21
22
23
24
25
31
32
33
40
41
42

Fig. 12

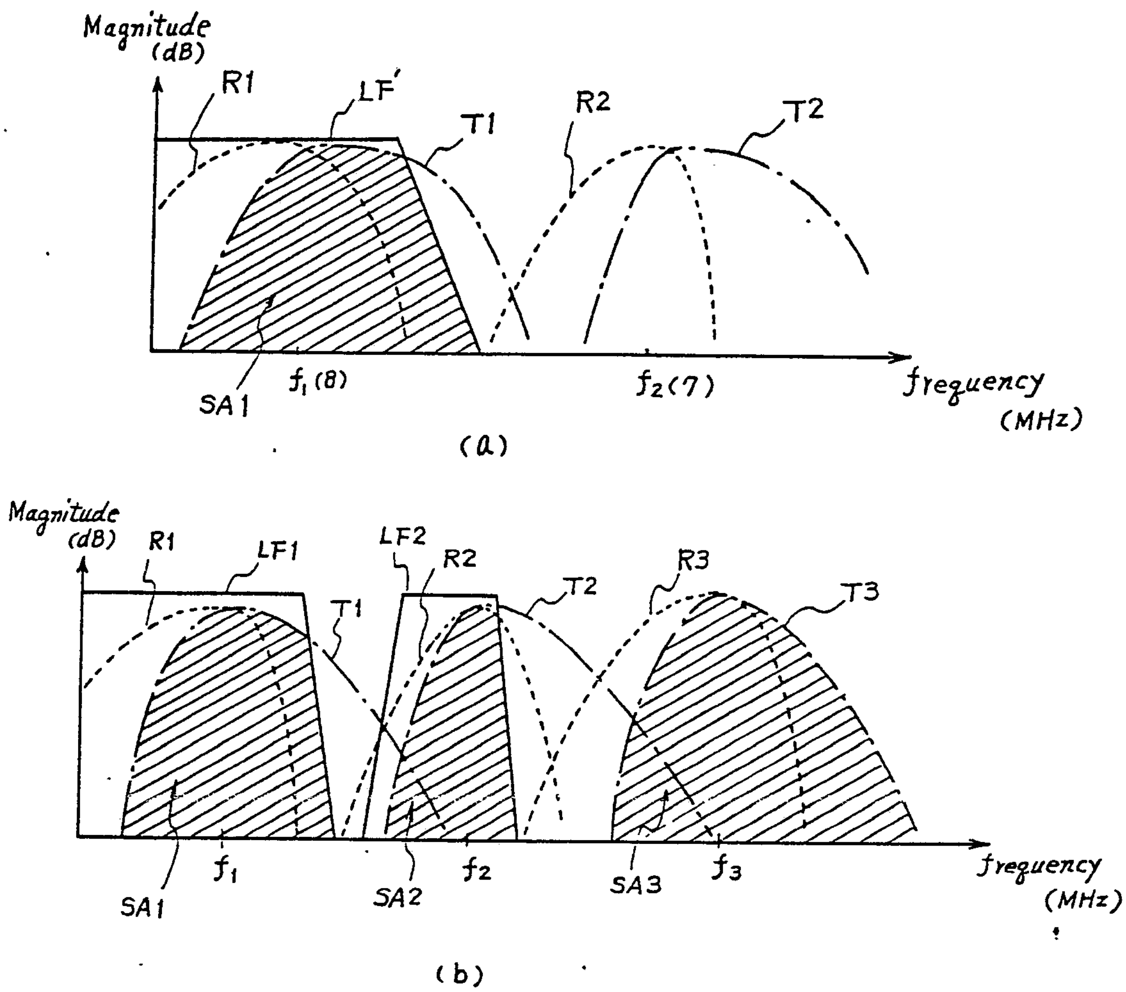
Magnitude (dB)
R1
LF'
T1
R2
T2
SA1
f1 (8)
f2 (7)
frequency (MHz)
(a)
Magnitude (dB)
R1
LF1
T1
LF2
R2
T2
R3
T3
SA1
f1
SA2
f2
SA3
f3
frequency (MHz)
(b)